# EUROPÄISCHE PATENTSCHRIFT

(11) **EP 3 503 739 B1**
(45) Veröffentlichungstag und Bekanntmachung des Hinweises auf die Patenterteilung: **09.10.2024**
(21) Anmeldenummer: 17754346.9
(22) Anmeldetag: 17.08.2017
(51) Int. Cl.: A23J 1/14, A23K 10/37, A23L 11/30, C12N 9/02

(54) **WERTPRODUKT UND VERFAHREN ZUR GEWINNUNG EINER WERTSTOFFPHASE**
VALUABLE PRODUCT AND METHOD FOR OBTAINING A VALUABLE MATERIAL PHASE
PRODUIT DE VALEUR ET PROCÉDÉ D'OBTENTION D'UNE PHASE DE MATIÈRE DE VALEUR

(30) Priorität: 26.08.2016 DE 102016115911
(43) Veröffentlichungstag der Anmeldung: 03.07.2019
(73) Patentinhaber: GEA Mechanical Equipment GmbH, 59302 Oelde (DE)
(72) Erfinder: HRUSCHKA, Steffen, 59302 Oelde (DE); BOSZULAK, Wladislawa, 59302 Oelde (DE); MARTEL, Daniel Michael, 74211 Leingarten (DE)
(74) Vertreter: Specht, Peter
(86) Internationale Anmeldenummer: PCT/EP2017/070848
(87) Internationale Veröffentlichungsnummer: WO 2018/036901

(56) Entgegenhaltungen:
- WO-A1-2015/155010
- WO-A1-2015/181203
- MARÍA ELENA CARTEA ET AL: "Phenolic Compounds in Brassica Vegetables", MOLECULES, vol. 16, no. 1, 30 December 2010 (2010-12-30), pages 251 - 280, XP055422152, DOI: 10.3390/molecules16010251
- NEDA NI&CACUTE;IFOROVI&CACUTE; ET AL: "Sinapic Acid and Its Derivatives: Natural Sources and Bioactivity : Sinapic acid and its derivatives…", COMPREHENSIVE REVIEWS IN FOOD SCIENCE AND FOOD SAFETY, vol. 13, no. 1, 17 December 2013 (2013-12-17), US, pages 34 - 51, XP055424560, ISSN: 1541-4337, DOI: 10.1111/1541-4337.12041
- YANZHOU ZHANG ET AL: "Hydrogen Peroxide-Resistant CotA and YjqC of Bacillus altitudinis Spores Are a Promising Biocatalyst for Catalyzing Reduction of Sinapic Acid and Sinapine in Rapeseed Meal", PLOS ONE, vol. 11, no. 6, 30 June 2016 (2016-06-30), pages e0158351, XP055423491, DOI: 10.1371/journal.pone.0158351
- M. NACZK ET AL: "Current research developments on polyphenolics of rapeseed/canola: a review", FOOD CHEMISTRY, vol. 62, no. 4, 1 August 1998 (1998-08-01), NL, pages 489 - 502, XP055423549, ISSN: 0308-8146, DOI: 10.1016/S0308-8146(97)00198-2
- LACKI K ET AL: "TRANSFORMATION OF 3,5-DIMETHOXY-4-HYDROXY CINNAMIC ACID AND ITS DERIVATIVES USING ENZYME FROM WHITE-ROT FUNGUS TRAMETES VERSICOLOR:ENZYME CHARACTERISTICS AND ITS APPLICATION", JOURNAL OF CHEMICAL TECHNOLOGY AND BIOTECHNOLOGY, WILEY, vol. 65, no. 3, 1 March 1996 (1996-03-01), pages 211 - 220, XP000581224, ISSN: 0268-2575
- YANZHOU ZHANG ET AL: "Hydrogen Peroxide-Resistant CotA and YjqC of Bacillus altitudinis Spores Are a Promising Biocatalyst for Catalyzing Reduction of Sinapic Acid and Sinapine in Rapeseed Meal", PLOS ONE, vol. 11, no. 6, 30 June 2016 (2016-06-30), pages e0158351, XP055423478, DOI: 10.1371/journal.pone.0158351
- YANXING NIU ET AL: "Characterization of the Factors that Influence Sinapine Concentration in Rapeseed Meal during Fermentation", PLOS ONE, vol. 10, no. 1, 21 January 2015 (2015-01-21), pages e0116470, XP055423476, DOI: 10.1371/journal.pone.0116470
- LACKI K ET AL: "Comparison of three methods for the determination of sinapic acid ester content in enzymatically treated canola meals", APPLIED MICROBIOLOGY AND BIOTECHNOLOGY, SPRINGER, BERLIN, DE, vol. 45, no. 4, 1 May 1996 (1996-05-01), pages 530 - 537, XP035171318, ISSN: 1432-0614, DOI: 10.1007/BF00578467
- ADINARAYANA KUNAMNENI ET AL: "Laccases and Their Applications: A Patent Review", RECENT PATENTS ON BIOTECHNOLOGY, vol. 2, no. 1, 1 January 2008 (2008-01-01), NL, pages 10 - 24, XP055422269, ISSN: 1872-2083, DOI: 10.2174/187220808783330965
- JOHANN F. OSMA ET AL: "Uses of Laccases in the Food Industry", ENZYME RESEARCH, vol. 2010, 1 January 2010 (2010-01-01), pages 1 - 8, XP055422279, DOI: 10.4061/2010/918761
- TIAN XIE ET AL: "Structural insight into the oxidation of sinapic acid by CotA laccase", JOURNAL OF STRUCTURAL BIOLOGY, vol. 190, no. 2, 1 May 2015 (2015-05-01), United States, pages 155 - 161, XP055422094, ISSN: 1047-8477, DOI: 10.1016/j.jsb.2015.03.005
- I. PARDO ET AL: "Re-designing the substrate binding pocket of laccase for enhanced oxidation of sinapic acid", CATALYSIS SCIENCE & TECHNOLOGY, vol. 6, no. 11, 1 January 2016 (2016-01-01), United Kingdom, pages 3900 - 3910, XP055424538, ISSN: 2044-4753, DOI: 10.1039/C5CY01725D

## Beschreibung

Die vorliegende Erfindung betrifft ein Wertprodukt in Form eines flavonoid-enthaltenden Phenolgemisches mit intensiver Rotfärbung und ein Verfahren zur Gewinnung einer Wertstoffphase, insbesondere einer rot gefärbten Phase, aus einem nativen Stoffgemenge.

Es ist bekannt, aus Saaten mit harten, zerbrechbaren Schalen, insbesondere aus Rapsfrüchten, eine Proteinphase als Wertstoffphase zu gewinnen.

Beim gängigen Ansatz zur Proteinkonzentratherstellung erfolgt eine Waschung der Schrote (stark entölt), wobei die löslichen Extraktionsstoffe abgereichert werden. Die Wertigkeit der entölten Zwischenprodukte hängt stark von der Konzentration an Begleitstoffen ab, wie Fasern, Zucker und sekundäre Pflanzenstoffen (Menner, M. u.a. "Fraktionierung pflanzlicher Rohstoffe zur simultanen Erzeugung von Lebensmitteln, technischen Rohstoffen und Energieträgern", Chemie Ingenieur Technik, Band 81, Ausgabe 11, Seiten 1743 - 1756, November 2009). Zu diesen Begleitstoffen zählen auch Polyphenole wie Sinapin. Die Polyphenolsäure "Sinapinsäure" kommt vor allem in Rapssamen vor (dort liegt der Sinapingehalt bei ca. 640 mg / 100 g Raps). Um Begleitstoffe wie Sinapin abzutrennen, werden große Verdünnungen gewählt, auch Proteine denaturiert (Temperatur, Alkohol), Zellulose wird enzymatisch abgebaut zu kurzkettigen Kohlenhydraten; diese Methoden werden gewählt, um die Stoffen besser extrahieren zu können.

Die Zugabe von Laccase zu Sinapinsäure ist u.a. aus verschiedenen wissenschaftlichen Publikationen bekannt:
LACKl K ET AL, "TRANSFORMATION OF 3,5-DIMETHOXY-4-HYDROXY CINNAMIC ACID AND ITS DERIVATIVES USING ENZYME FROM WHITE-ROT FUNGUS TRAMETES VERSICOLOR:ENZYME CHARACTERISTICS AND ITS APPLICATION", JOURNAL OF CHEMICAL TECHNOLOGY AND BIOTECHNOLOGY, WILEY, (19960301), vol. 65, no. 3, ISSN 0268-2575;
YANZHOU ZHANG ET AL, "Hydrogen Peroxide-Resistant CotA and YjqC of Bacillus altitudinis Spores Are a Promising Biocatalyst for Catalyzing Reduction of Sinapic Acid and Sinapine in Rapeseed Meal", PLOS ONE, (20160630), vol. 11, no. 6, doi:1 0.1 371/journal.pone.01 58351;
YANXING NIU ET AL, "Characterization of the Factors that Influence Sinapine Concentration in Rapeseed Meal during Fermentation", PLOS ONE, (20150121), vol. 10, no. 1, doi:10.1371/journal.pone.0116470;
LACKl K ET AL, "Comparison of three methods for the determination of sinapic acid ester content in enzymatically treated canola meals", APPLIED MICROBIOLOGY AND BIOTECHNOLOGY, SPRINGER, BERLIN, DE, (19960501), vol. 45, no. 4, doi:10.1007/BF00578467, ISSN 1432-0614;
ADINARAYANA KUNAMNENI ET AL, "Laccases and Their Applications: A Patent Review", RECENT PATENTS ON BIOTECHNOLOGY, NL, (20080101), vol. 2, no. 1, doi:10.2174/187220808783330965, ISSN 1872-2083;
JOHANN F. OSMA ET AL, "Uses of Laccases in the Food Industry", ENZYME RESEARCH, (20100101), vol. 2010, doi:10.4061/2010/918761;
TIAN XIE ET AL, "Structural insight into the oxidation of sinapic acid by CotA laccase", JOURNAL OF STRUCTURAL BIOLOGY, United States, (20150501), vol. 190, no. 2, doi:10.1016/j.jsb.2015.03.005, ISSN 1047-8477;
PARDO ET AL, "Re-designing the substrate binding pocket of laccase for enhanced oxidation of sinapic acid", CATALYSIS SCIENCE & TECHNOLOGY, United Kingdom, (20160101), vol. 6, no. 11, doi:10.1039/C5CY01725D, ISSN 2044-4753;

Vor diesem Hintergrund ist es die Aufgabe der Erfindung, ein intensiv-rot gefärbtes Wertprodukt zu gewinnen und die Gewinnung von Wertprodukten aus dem nativen Stoffgemenge weiter zu optimieren, wobei es insbesondere möglich sein soll, auf relativ einfache Weise eine intensiv rot gefärbte Wertstoffphase aus dem nativen Stoffgemenge zu gewinnen.

Die Erfindung löst diese Aufgabe durch das Bereitstellen eines Verfahrens mit den Merkmalen des Anspruchs 1 und durch ein Wertprodukt mit den Merkmalen des Anspruchs 11.

Ein Wertprodukt ist ein flavonoid-enthaltendes Phenolgemisch.

Dieses flavonoid-enthaltende Phenolgemisch umfasst ein Reaktionsprodukt. Dieses ist vorzugsweise eine Phenolische Verbindung. Besonders bevorzugt kann es sich bei der phenolischen Verbindung um ein Flavonoid handeln. Das Reaktionsprodukt wird gebildet bei der Zugabe von Laccase zu einer sinapinsäurehaltigen wäßrigen und/oder alkoholischen Phase. Diese alkoholische oder wäßrige Phase ist erfindungsgemäß hergestellt aus Pflanzen und/oder Pflanzenteilen, vorzugsweise aus Saaten und/oder Früchten von Kreuzblütengewächsen (Brassicaceae), insbesondere von Rapsfrüchten oder Camelina. Die Bildung des Reaktionsproduktes erfolgt unter Anwesenheit von Sauerstoff.

Als "sinapinsäurehaltig" werden in Rahmen der vorliegenden Erfindung auch Sinapinsäurederivate verstanden, so z.B. Sinapinsäureester.

Die Dimerisierung von Sinapinsäure mit Laccase in Anwesenheit von Sauerstoff unter Bildung eines roten Farbstoffes ist an sich bekannt. Es hat sich allerdings überraschend gezeigt, dass die Rotfärbung, bei der Verwendung einer sinapinsäurehaltigen Phase, welche aus Pflanze oder Pflanzenteilen gewonnen wurde, gegenüber einer Färbung des Laccase-Sinapinsäure-Reaktionsproduktes deutlich intensiver ist. Dies kann ggf. auf die Anwesenheit weiterer Reaktionspartner in der alkoholisch-wäßrigen Phase zurückzuführen sein, welche bei der Reaktion der beiden reinen bzw. isolierten Reaktionspartner nicht vorhanden sind.

Vorteilhafte Ausgestaltungen der Erfindung sind Gegenstand der Unteransprüche.

Das Reaktionsprodukt kann vorteilhaft in einer wäßrigen und/oder alkoholischen Lösung und/oder Dispersion vorliegen. Die Lösung und/oder Dispersion ist in diesem Fall das Wertprodukt. Das Reaktionsprodukt ist vorzugsweise eine Phenolische Verbindung und besonders bevorzugt ein Flavonoid.

Das Wertprodukt, also das flavonoid-enthaltende Phenolgemisch, weist einen Trockensubstanzgehalt von mehr als 55 % auf. Ein derart erhöhter Trockensubstanzgehalt erhöht die Stabilität der Lösung, so dass sich das Reaktionsprodukt langsamer oder gar nicht zersetzt.

Als wäßrige Lösung wird im Rahmen der vorliegenden Erfindung auch ein Gemisch aus Wasser und einem organischen wasserlöslichen Lösungsmittel verstanden. Dieses organische wasserlösliche Lösungsmittel kann ein Alkohol, insbesondere ein Alkohol mit drei oder weniger Kohlenstoffatomen, und besonders bevorzugt Ethanol, sein.

Eine alkoholische Lösung besteht demgegenüber ausschließlich aus Alkohol, insbesondere aus einem Alkohol mit drei oder weniger Kohlenstoffatomen, und besonders bevorzugt aus Ethanol.

Die sinapinsäurehaltige wäßrige und/oder alkoholische Phase als Ausgangsstoff ist vorteilhaft aus kaltgepressten Saaten und/oder Früchten hergestellt.

Der pH-Wert der wäßrigen und/oder alkoholischen Phase beträgt vorzugsweise pH=7 oder weniger.

Die wäßrige und/oder alkoholische Phase weist vorteilhaft einen Trockensubstanzgehalt vor der Zugabe von Laccase von weniger als 3%, vorzugsweise weniger als 1 % auf.

Das vorgenannte Wertprodukt wird erfindungsgemäß nach dem folgenden Verfahren hergestellt.

Ein erfindungsgemäßes Verfahren zur Gewinnung einer Wertstoffphase, insbesondere eines erfindungsgemäßen Wertproduktes aus einem nativen Stoffgemenge, umfasst die folgenden Schritte:
- Schritt A: Bereitstellen des nativen Stoffgemenges aus Saaten von Kreuzblütengewächsen (Brassicaceae), mit einem Anteil von harten, zerbrechbaren Schalen oder in geschälter Form, insbesondere von Rapssaaten als Stoffgemenge aus den vollständigen Saaten oder aus bereits (teil-) entölten Saaten, insbesondere als Presskuchen, der bei einem Abpressen von Öl insbesondere mit einer Presse als Rückstand der Ölgewinnung verbleibt;
- Schritt B: sofern das Stoffgemenge aus Schritt A noch nicht zerkleinert ist: Zerkleinern des Stoffgemenges, wobei ggf. die Schalen aufgebrochen werden;
- Schritt C: Dispergieren des zerkleinerten Stoffgemenges aus Schritt A) oder B) mit Wasser, wobei auf einen Teil zerkleinertes Stoffgemenge vorzugsweise bis zu maximal 8, besonders vorzugsweise bis zu maximal 6, insbesondere bis zu maximal 5 Teile Wasser zugegeben werden und wobei das Wasser und das zerkleinerte Stoffgemenge gerührt werden, so dass sich ein fließfähiger Brei bzw. eine Dispersion ergibt;
- Schritt D): Einstellen des pH-Wertes des Breis aus Schritt C) in einen alkalischen Bereich pH > 9, 5;
- Schritt E): Zugeben eines wasserlöslichen organischen Lösemittels, vorzugsweise von Ethanol, insbesondere in mit Wasser verdünnter Form, zu dem Brei aus Schritt D) im Anschluss an das Einstellen des pH-Wertes des Breis im Schritt D; insbesondere derart, dass eine Alkoholkonzentration erreicht wird, die kleiner als 30% ist, um die Schalen vom Endosperm der Saaten/Früchte zu lösen;
- Schritt F): Abtrennen einer Feststoffphase, welche den überwiegenden Anteil der ggf. noch vorhandenen Schalen aufweist, vorzugsweise in einer Zentrifuge im Zentrifugalfeld;
- Schritt G): Verschieben des pH-Wertes des Feststoffphase befreiten Breis aus Schritt F) in den pH-Bereich von pH = 4,5 bis pH = 7,2; und
- Schritt H): Trennen des schalenfreien Breis, dessen pH-Wert in Schritt G) ins Saure verschoben worden ist, vorzugweise in einer Zentrifuge, insbesondere in wenigstens einem Dekanter oder einem Separator, in mehrere Phasen, wobei zumindest eine dieser Phasen eine Polyphenol-Albumin-Flüssigkeitsphase ist;
- Schritt I): Zugabe von Laccase zu der Polyphenol-Albumin-Flüssigkeitsphase des Schrittes H) direkt oder nach einem Durchlaufen weiterer Zwischenschritte.

Die Polyphenol-Albumin-Flüssigkeitsphase entspricht der vorgenannten sinapinsäurehaltigen wäßrigen und/oder alkoholischen Phase, wobei das Polyphenol die Sinapinsäure oder ein Sinapinsäurederivat ist.

Das erfindungsgemäße Verfahren stellt eine besonders wirtschaftliche Gewinnung des vorgenannten Wertproduktes mit intensiver Rotfärbung dar.

Vorteilhafte Ausgestaltungen des Verfahrens sind Gegenstand der Unteransprüche.

Die Polyphenol-Albumin-Flüssigkeitsphase des Schrittes I), der Laccase zugesetzt worden ist, nimmt dabei nach einer Reaktionszeit von maximal 30 min eine rote Färbung an.

Der Polyphenol-Albumin-Flüssigkeitsphase des Schrittes H) im Schritt I) wird vorteilhaft Laccase in Anwesenheit von Sauerstoff in folgender Menge zugesetzt: zumindest 0,1 g/l, vorzugsweise 0,15 bis 0,25 g/l an Laccase bezogen auf eine Enzymaktivität von 0,28 Kilounits , Dabei erfolgt die Bildung des erfindungsgemäßen Reaktionsproduktes.

Im Schritt H) kann vorteilhaft folgende Phasentrennung in einem oder zwei Schritten, vorzugsweise in einer Zentrifuge, insbesondere in einem Dekanter oder Separator erfolgen:
- ölhaltige Phase mit Triglyceridgehalt;
- wässrige Phase mit Albumin und Sinapinsäuregehalt; und
ggf. eine dritte Phase mit einem weiteren Wertprodukt.

Im Schritt H) kann vorteilhaft zudem folgende Phasentrennung in einem oder zwei Schritten, vorzugsweise in einer Zentrifuge, insbesondere in einem Dekanter oder Separator, in zwei Wertstoffphasen erfolgen, mit zumindest einer wässrigen Phase mit Albumingehalt und Sinapinsäuregehalt und Restölgehalt.

Als Stoffgemenge/Ausgangsmaterial kann ein "kurz zuvor hergestelltes Zwischenprodukt" verarbeitet werden, d.h. nach der Vorstufe sind nicht mehr als 31 Tage vergangen.

Als Stoffgemenge/Ausgangsmaterial kann ein "frisches Zwischenprodukt" verarbeitet werden, d.h. nach der Vorstufe dürfen nicht mehr als 3 Tage vergangen sein, vorzugsweise sogar nur weniger als 48 Stunden, insbesondere weniger als 24 Stunden.

Als Stoffgemenge kann in Schritt A kalt gepressten Material, insbesondere ein kalt gepresster Rapspresskuchen verwendet wird, der bei einer Temperatur kleiner als 70°C, besonders vorzugsweise sogar kleiner als 60°C, gepresst worden ist
Einer oder mehrere der Abtrennungsschritte kann oder können vorteilhaft in einem 3-Phasendekanter oder in zumindest zwei Schritten in 2-Phasendekantern erfolgen.

Einer oder mehrere der Abtrennungsschritte kann oder können vorteilhaft in einem Düsenseparator erfolgen.

Das wasserlösliche organische Lösemittel kann vorteilhaft ein linearer aliphatischer Alkohol sein.

Der Gehalt an wasserlöslichem organischen Lösemittel im wässrigen Anteil des Breis (I) nach dem Zugeben des wasserlöslichen organischen Lösemittels kann vorteilhaft weniger als 45 Vol. %, vorzugsweise weniger als 30 Vol % und besonders vorzugsweise weniger als 15 Vol %, betragen.

Die Temperatur kann vorteilhaft während sämtlicher Verfahrensschritte, wozu nicht das Pressen zur Erzeugen des Presskuchens gehört, unterhalb von 60 ° C liegen.

Die Temperatur kann vorteilhaft während sämtlicher Verfahrensschritte, wozu nicht das dem Verfahren vorangehende Pressen zur Erzeugen eines Presskuchens als Ausgangsmaterial gehört, unterhalb von 50 ° C liegen.

Besonders bevorzugt werden abgesehen von der Laccase keine weiteren Enzyme oder andere Chemikalien (außer zur pH-Wertanpassung) in den Schritten A) bis I) und im gesamten Herstellverfahren zugegeben.

Weiterhin erfindungsgemäß ist ein Erzeugnis, welches durch das erfindungsgemäße Verfahren hergestellt ist.

Nachfolgend wird der Gegenstand der Erfindung anhand eines Ausführungsbeispiels und unter Bezugnahme auf die beiliegende Figur näher erläutert. Es zeigt:

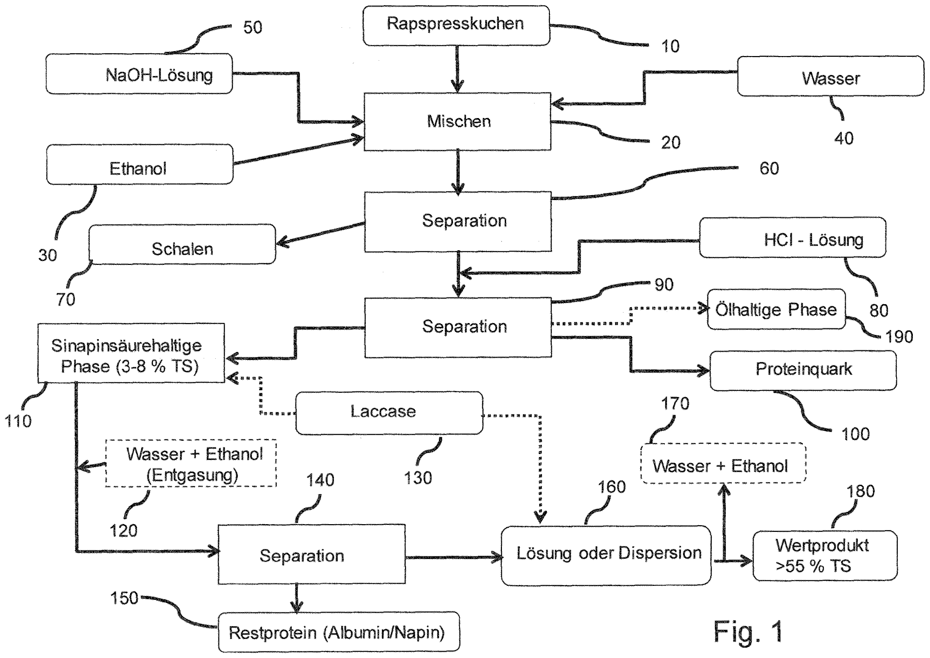

- Fig. 1: Ablaufdiagramm eines Ausführungsbeispiels zur Durchführung eines erfindungsgemäßen Verfahrens zur Herstellung eines erfindungsgemäßen Wertproduktes.

Fig. 1 stellt schematisch einen beispielhaften Verfahrensablauf für die Gewinnung eines Wertproduktes mit intensiver Rotfärbung dar. Diese Rotfärbung bleibt auch über einen längeren Zeitraum bei Lagerung unter Raumtemperatur gut erhalten.

Die Zugabe der Laccase 130 kann vorzugsweise an einer der beiden zwei Stellen erfolgen. Die beiden Optionen sind mit gestichelten Pfeilen dargestellt.

Das erfindungsgemäße Verfahren weist vorzugsweise folgende Schritte auf:

### Schritt A)

Als Ausgangsmaterial wird natives Stoffgemenge aus Saaten (Samen) mit harten, zerbrechbaren Schalen, insbesondere aus
- Saaten/Früchten von Kreuzblütengewächsen (Brassicaceae), insbesondere von Rapsfrüchten oder Camelina wie z.B. Leindotter, bereitgestellt.

Das Stoffgemenge im Sinne dieser Anmeldung kann aus den vollständigen, jedoch gebrochenen Saaten bestehen. Diese können ungeschält sein oder teilweise geschält oder ganz geschält.

Alternativ kann das Stoffgemenge aber auch aus einem bereits entölten Produkt bestehen, insbesondere aus einem "Zwischenprodukt", d.h. aus einem Presskuchen 10, der nach einer "Vorstufe", z.B. dem Abpressen von Öl, insbesondere mit einer Presse (z.B. einer Schneckenpresse) als Rückstand der Ölgewinnung verbleibt.

Besonders bevorzugt wird als das Ausgangsmaterial "kurz zuvor gewonnenes Zwischenprodukt" verarbeitet, d.h. nach der Vorstufe dürfen nicht mehr als 31 Tage vergangen sein.

Die Saat kann frisch geerntet oder aber Tage, Wochen oder Monate alt sein, die Zwischenstufe (das Pressen) sollte kurz oder sogar unmittelbar vor der weiteren Verarbeitung stattfinden, damit sich nach der Ölgewinnung das Material - die Saat - nicht zu stark verändert hat.

Ganz bevorzugt wird als das Ausgangsmaterial "frisches Material" verarbeitet, d.h. nach eine Vorstufe bzw. Vorbearbeitung (Ölgewinnen) dürfen nicht mehr als 3 Tage vergangen sein, vorzugsweise sogar nur weniger als 48 Stunden oder 24 Stunden oder 12 h oder weniger als 1h.

Mit Material aus einem Zeitraum kurz nach der Vorstufe werden gute oder/und mit frischem Material in der Regel nochmals bessere Ergebnisse hinsichtlich der Ausbeute und der Reinheit der Wertprodukte erzielt.

Der Presskuchen 10 kann einen Restölgehalt aufweisen, der auch bei 20% oder mehr liegen kann. Trotz derart hoher Restölgehalte ist die Gewinnung auch einer Proteinphase mit der Erfindung auf einfache Weise realisierbar. Die Proteingewinnung ist dabei jedoch lediglich optional. Die Sinapinsäure und/oder das Sinapinsalz als mögliches Sinapinsäurederivat kann somit als einziges Wertprodukt des Stoffgemenges gewonnen werden oder als zusätzliches Wertprodukt bei einer Proteingewinnung gewonnen werden.

### Schritt B)

Sofern es noch nicht zerkleinert vorliegt: Zerkleinern des Stoffgemenges aus Schritt a) zum Aufbrechen der Schalen. Sofern ein Presskuchen verwendet wird, wird dieser aufgebrochen, idealerweise unmittelbar nach dem Pressen, noch warm. Derart wird ein zerkleinertes Material - eine Art Granulat - aus dem Presskuchen erzeugt. Das zuvor durch einen Pressvorgang (teil-) entölte Stoffgemenge wird in der Regel nur zerkleinert, beispielsweise zerbröselt bzw granuliert oder es werden jedenfalls die Schalen aufgebrochen.

### Schritt C)

Das bereitgestellte und zerkleinerte Stoffgemenge aus Schritt A) oder B) wird durch Mischen 20 mit Wasser 40 und/oder einer wässrigen Lösung (z.B. einer Salzlösung) dispergiert. Auf einen Teil "zerkleinertes Produkt" werden vorzugswiese bis zu maximal 8, vorzugsweise bis zu maximal 5 Teile (Gewichtsanteile) Wasser zugegeben. Sodann werden Wasser und zerkleinertes Produkt gerührt, so dass sich ein fließfähiger Brei bzw. eine Dispersion ergibt. Das Rühren erfolgt vorzugsweise für mehr als 30 min, insbesondere für mehr als 1h.

### Schritt D)

Als nächstes erfolgt ein Einstellen des pH-Wertes des Breis (I) aus Schritt c) in einen alkalischen Bereich; Vorzugsweise wird der pH-Wert des Breis bzw. der Dispersion mit Lauge 50 auf pH 10 bis 11 eingestellt. Dabei wird (vorsichtig) das Rühren fortgesetzt. Die Verrührzeit beträgt vorzugsweise mehr als 30 min, vorzugsweise liegt sie bei 1 h oder darüber.

### Schritt E)

In diesem weiteren Schritt erfolgt ein Zugeben mindestens eines wasserlöslichen organischen Lösemittels, vorzugsweise von Alkohol 30, insbesondere in mit Wasser verdünnter Form im Anschluss an das Einstellen des pH-Wertes des Breis im Schritt D. Vorzugsweise wird die Dispersion, deren pH-Wert in den alkalischen Bereich eingestellt worden ist, mit dem Alkohol Ethanol bzw. EtOH (vorzugsweise 30-60 Vol.%) auf eine Alkoholkonzentration von 20-15 Vol.% oder weniger, insbesondere 12% EtOH Konzentration gebracht. Entsprechend der Wassermenge des verwendeten Alkohols kann die Wassermenge in Schritt C um das im Alkohol, insbesondere im 30-60%igen EtOH enthaltene Wasser, reduziert werden. Damit lösen sich die Schalen vom Endosperm (Kotyledon) mit dem Restöl und können abgetrennt werden, insbesondere zentrifugal.

Als weniger bevorzugte Alternative gegenüber Ethanol können auch andere Alkohole, so z.B. Isopropanol, verwandt werden.

Die Schritte C-E können gemeinsam, beispielsweise zeitgleich, erfolgen. So ist es z.B. möglich eine wässrige stark verdünnte Ethanollösung mit Lauge auf einen entsprechend basischen pH-Wert einzustellen und diese Lösung dem zerkleinerten Stoffgemenge zuzugeben.

### Schritt F)

Im Schritt F) erfolgt daher bei einem ersten Separieren 60 ein Abtrennen einer Feststoffphase, welche den überwiegenden Anteil, vorzugsweise mehr als 80 Gew.%, der Schalen 70 umfasst, vorzugsweise in einer Zentrifuge im Zentrifugalfeld aus dem Brei bzw. es erfolgt ein Klären des Breis von Schalen-Feststoffanteilen insbesondere in einem Dekanter.

Bei diesem Schritt werden mit einem Dekanter mit einem Zulauf die Schalen von dem Restbrei getrennt.

Die leichtere Phase einer zentrifugalen Phasentrennung wird nachfolgend auch gelegentlich als Oberlauf bezeichnet und die Feststoffphase als schwere Phase. Eine Mittelphase läge entsprechend bzgl. ihrer Dichte dazwischen.

### Schritt G)

Der jedenfalls weitestgehend schalenfreie Brei aus Schritt F) wird nunmehr weiterverarbeitet. Vorzugsweise erfolgt dabei eine Ausfällung des gelösten - Proteinanteiles aus dem schalenfreien Brei, der zusammen mit dem un- oder angelösten Protein-Teil eine Fraktion bildet, den Quark. Der pH-Wert wird dabei wieder weiter in den sauren Bereich verschoben, insbesondere in den pH-Bereich von pH = 4,5 bis pH = 7. Hierzu kann Salzsäure 80, vorzugsweise in verdünnter Form, zugegeben werden.

### Schritt H)

Sodann wird der schalenfreie Brei, dessen pH-Wert wieder ins Saure verschoben worden ist, durch ein zweites Separieren 90 - vorzugweise in einer Zentrifuge, insbesondere in wenigstens einem Dekanter oder in einem Separator - in einem oder zwei Schritten in Wertstoffphasen getrennt, von denen eine Phase eine Proteinkonzentratphase ist und eine dieser Phasen eine sinapinsäurehaltige Flüssigkeitsphase ist;

Besonders bevorzugt erfolgt eine Trennung in folgende zwei oder drei Phasen:
- ölhaltige Phase 190 welche als optionale separate Phase mit gestrichelten Pfeilen dargestellt ist
- wässrige Phase (Sinapinsäurehaltig) 110
- Proteinkonzentratphase (nachfolgenden auch "Proteinquark" genannt) 100 oder
- wässrige sinapinsäurehaltige Phase mit Restölgehalt 110; und
- Proteinkonzentratphase (Proteinquark) 100;

In der wäßrigen Phase können weitere gelöste Pflanzenbestandteile vorhanden sein. Dabei handelt es sich unter anderem um eines oder mehrere Albumine und/oder Polyphenole und/oder weitere in den o.g. Pflanzen enthaltene pflanzliche Bestandteile, welche nicht unter den o.g. Bedingungen als Feststoffe abgetrennt wurden.

Die Zwei-Phasentrennung wird dann ausgeführt, wenn das Rohmaterial relativ stark entölt ist und/oder im Feststoff gebunden vorliegt oder wenn kein intensiver Scherungseinfluss ausgeführt worden ist. Die Zugabe von Wasser oder Alkohol oder Lauge oder dgl. kann auch in Teilschritten erfolgen. Das Öl als leichtere Phase enthält Triglyceride und ist einer der gewinnbaren Wertstoffe.

Schritt I) Zugabe von Laccase 130 zur sinapinsäurehaltigen Flüssigkeitsphase des Schrittes H) mit oder ohne Restölgehalt direkt oder nach einem Durchlaufen weiterer Zwischenschritte. Die sinapinsäurehaltige Flüssigkeitsphase 110 des Schrittes I), der Laccase 130 zugesetzt worden ist, nimmt nach einer Reaktionszeit von vorzugsweise weniger als 30 min eine rote Färbung an.

Nachfolgend wird zumeist von der Sinapinsäure und sinapinsäurehaltigen Phasen und Stoffgemengen gesprochen. Es versteht sich allerdings, dass je nach pH-Wert die Sinapinsäure auch als Sinapinsäurederivat z.B. verestert mit Cholin vorliegen kann. Die Sinapinsäure kann somit auch als Sinapinsäurederivat in der jeweiligen Phase vorliegen. Diese Verbindungen werden ebenfalls durch die Begriffe "Sinapinsäure" oder "sinapinsäurehaltig" erfasst.

Vorzugsweise liegt die Temperatur während sämtlicher Verfahrensschritte unter 60°C, insbesondere unter 50°C, vorzugsweise, zwischen 40 °C und 50°C, wodurch sich besonders wertvolle Produkte gewinnen lassen.

Die Denaturierung der Proteine ist ein temperatur- und zeitabhängiger Prozess. Hinzu kommt die Bedingung im alkoholischen Milieu. Die Proteindenaturierung geht umso schneller, je höher die Temperatur ist. In wässriger Umgebung ist aber auch bei Wärmeinwirkungen von 45-50 °C keine irreversible Proteindenaturierung zu erwarten. Das ändert sich aber mit der Alkoholkonzentration. Schon bei Umgebungstemperatur ist bei hochkonzentriertem Alkohol eine Proteinausfällung zu beobachten.

Je geringer nun die Alkoholkonzentration ist, umso höher muss die Temperatur sein, um die Proteine zu denaturieren. Oder umgekehrt: je wässriger die Alkoholkonzentration ist, umso höher darf die Arbeitstemperatur sein, ohne dass die Proteine irreversibel geschädigt werden.

Man wird also (für reines Wasser) eine möglichst hohe, d.h. möglichst an 60°C heranreichende Temperatur wählen, um möglichst viele Stoffe in Lösung zu bringen, wie Proteine, Lecithine, Glycolipide etc.. Es ist jedoch darauf zu achten, dass die Temperatur entsprechend den Prozessparametern Zeit und Alkoholkonzentration (ggf. Druck) hinreichend niedrig bleibt.

Die gefällten Proteine aus Schritt H) liegen als Proteinquark vor (schwere Phase). Sie bilden einen weiteren der gewinnbaren Wertstoffe. Diese Phase kann gut zu Pulver getrocknet werden.

Nach dem Schritt I) wird nach dem Verstreichen einer genügenden Reaktionszeit ein auch optisch ansprechendes und daher gut weiter verwertbares Fluid roter Färbung gewonnen. Das Fluid weist eine Färbung auf, die der Farbe der Frucht "rote Beete" ähnelt. Als RAL-Farbe werden normierte Farben bezeichnet (RAL GmbH, Tochter des RAL-Institutes). Jeder Farbe ist eine vierstellige Farbnummer zugeordnet. Theoretisch kann für das Verfahren jeder Presskuchen verwendet werden.

Die vorteilhafte Temperaturangabe zu den Verfahrensschritten A bis H bezieht sich nicht auf die Presstemperatur beim Erzeugen des Presskuchens bei der Ölerzeugung. Je höher die Temperatur bei den vorangegangen Prozessschritten war, umso brauner wird die Proteinphase bzw. Quarkfraktion. Dies liegt einerseits an der Maillard-Reaktion von Zuckern mit Proteinen, andererseits an der Phenoloxidation. Gegenüber der DE 10 2011 050 905 A1 wird insbesondere durch die Verwendung optimiert ausgewählten Ausgangsmaterials (vorzugsweise kalt gepresster Raps-Presskuchen, vorzugsweise sehr frisch) ein besonders ansprechendes, besonders gut weiterverwertbares Produkt gewonnen.

Besonders vorteilhaft ist die Verwendung kalt gepressten Materials, insbesondere eines kalt gepressten Rapspresskuchens (Temperatur beim Pressen vorteilhaft kleiner als 70°C, besonders vorzugsweise sogar kleiner als 60°C) als Ausgangsmaterial bzw. als das bereitgestellte Stoffgemenge. Warm gepresstes Material wird beim Pressen deutlich höheren Temperaturen (bis über 100° C) ausgesetzt. Durch die Verwendung kalt gepressten Materials als Ausgangsmaterial für das erfindungsgemäße Verfahren kann eine Proteinphase bzw. "Protein- bzw. Quarkphase" mit deutlich besseren Eigenschaften (insbesondere hinsichtlich der Farbe deutlich heller und daher besser verarbeitbar) und mit deutlich besserer Ausbeute gewonnen werden als bei der Verwendung warm bzw. heiß gepressten Ausgangsmaterials. Dies wurde im Stand der Technik bisher nicht erkannt. Denn gängige Rapspressverfahren zielen auf eine hohe Ölausbeute, weshalb beim Pressen gern höhere Temperaturen verwendet werden. Als Nebeneffekt ist festzustellen, dass Sinapin (ein Polyphenol) abgebaut wird, was an sich vorteilhaft für die Proteinfraktion wäre. Beim erfindungsgemäßen Verfahren stellt der originale, also nicht reduzierte, Sinapingehalt im kalt gepressten Kuchen aber dennoch kein Problem für das Endprodukt dar, da die polyphenolischen Verbindungen im Wesentlichen nicht in der Quarkphase zu finden sind, da sie in die Wasserphase übergehen.

In der Flüssigkeitsphase bzw. "Wasserphase" des Schrittes H) sind noch wertvolle Inhaltsstoffe enthalten, insbesondere ist sie relativ stark albuminhaltig und/oder sinapinsäurehaltig. Auch ist die Flüssigphase mit Sinapin, Sinapinsäure und/oder Sinapinsäurederivate angereichert, Sinnvoll und vorteilhaft ist insofern die erfindungsgemäße Gewinnung eines rot gefärbten Fluids.

Im Anschluss an Schritt I) kann in einem Schritt K) eine Entgasung 120 zur teilweisen Entfernung von Wasser und/oder Ethanol, vorzugsweise auf temperaturschonende Weise, erfolgen. Dies kann vorteilhaft durch Vakuum oder Unterdruck erfolgen. Dies ist besonders vorteilhaft je höher der Ethanolgehalt in der wässrigen Phase ist.

Schließlich kann in Schritt L) eine optionale Abtrennung einer Restproteinphase 150 durch eine dritte Separation 140 erfolgen. In der Restproteinphase 150 sind insbesondere Albumine und/oder Napine enthalten, welche als Feststoffe durch einen Separator, insbesondere durch einen Dekanter abtrennbar sind. Dies kann ggf. unter Zugabe von Additiven, wie z.B. Enzymen; Komplexbildnern und/oder Fällungsmitteln, wie z.B. Ammoniumsulfat erfolgen.

Die Laccase gemäß Schritt I) kann sowohl vor als auch nach den optionalen Schritten K und/oder L zugegeben werden. Die Reaktion der Sinapinsäure und/oder des Sinapinsäurederivats mit der Laccase erfolgt in Schritt I) besonders bevorzugt in Anwesenheit mit Sauerstoff erfolgen. Bevorzugt kann zusätzlich Sauerstoff, beispielsweise durch Umluftbetrieb, während der Reaktion eingeleitet werden.

An der Reaktion ist Sinapinsäure bzw. ein Sinapinsäurederivat und Laccase beteiligt. Es kann jedoch auch noch eine oder mehrere weitere Substanzen beteiligt sein, welche gelöst in der wässrig-alkoholischen Phase während der vorhergehenden Schritte A-H, und vorzugsweise auch in den optionalen Schritten K und L mitgeführt werden.

Das hergestellte Reaktionsprodukt aus Sinapinsäure, Laccase und ggf. weiteren Reaktionsprodukten ist mit einem Trockensubstanzgehalt von 3-8% in der wäßrigalkoholischen Lösung 160 enthalten.

Es wurde überraschend gefunden, dass die Rotfärbung bei der Zugabe von Laccase zu einer sinapinsäurehaltigen Phase, welche bei der Verarbeitung von Pflanzen, insbesondere von Brassicaceae, anfällt, deutlich intensiver ausfällt als die Rotfärbung welche bei der Reaktion von isolierter bzw. reiner Sinapinsäure mit Laccase, wie sie z.B. im Artikel "Transformation of 3,5-Dimethoxy 4-hydroxy Cinnamic Acid by Polyphenol Oxidase from Fungus Trametes versicolor" (Lacki and Duvnjak, Biotechnology and Bioengineering, Vol. 57, No. 6, p.694-703) beschrieben wurden.

In einem weiteren Schritt M erfolgt schließlich eine Stabilisierung des Reaktionsproduktes mit der intensiven Rotfärbung. Dies erfolgt durch Aufkonzentrierung des Reaktionsproduktes durch Entfernen von Wasser und Alkohol 170, z.B. durch Entgasen bei Unterdruck oder Vakuum. Die Aufkonzentrierung kann entweder bereits im Schritt 120 vor dem Abtrennen der Albumine erfolgen oder erst im Schritt 170 oder aber durch ein erstes Aufkonzentrieren in Schritt 120 und ein zweitens Aufkonzentrieren im Schritt 170. Es bildet sich ein roter Sirup als Wertprodukt 180 mit einem Trockensubstanzgehalt von zumindest 20 Prozent, vorzugsweise 30-35 Prozent. Ein Teil des Sirups ist dabei allerdings vorzugsweise noch Wasser und/oder Ethanol. Weiterhin kann das Wertprodukt, also der Sirup Restmengen an Sinapinsäure enthalten, welche jedoch unter 500 ppm, vorzugsweise unter 400 ppm liegen. Damit ist das Wertprodukt direkt in der Lebensmittelindustrie, z.B. als Farbstoff zum Einfärben von Lebensmitteln, verwendbar.

Bei dem vorgenannten Wertprodukt handelt es sich vorzugsweise um ein flavonoidhaltiges Phenolgemisch. Dabei ist das Reaktionsprodukt mit der roten Farbe eine Phenolische Verbindung, insbesondere ein Flavonoid.

Der Großteil der Sinapinsäure ist dabei vorzugsweise zum Reaktionsprodukt bei Laccasezugabe umgesetzt. So sind weniger Mol% Sinapinsäure als Reaktionsprodukt im Sirup enthalten. Besonders bevorzugt kann der Stoffmengenanteil an Sinapinsäure gegenüber dem Reaktionsprodukt bei weniger als 50 % beträgen. Das heißt, dass bevorzugt zwei Drittel der Sinapinsäure umgesetzt wurden.

Je nach Konzentrationsbereich und pH-Wert kann es zu einer Stabilisierung, einem langsamen oder einem schnellen Zersetzen des Farbstoffes bzw. des Reaktionsproduktes kommen. Daher kommen verschiedene Anwendungen für das rote Reaktionsprodukt in Betracht.

Eine Lösung mit einer Konzentration des Reaktionsproduktes von mehr als 20 % kann sowohl bei normaler Umgebungstemperatur von 20-35°C oder auch kühl gelagert werden.

Die Zersetzung des Reaktionsproduktes ist u.a. pH-wertabhängig. So ist es z.B. denkbar, das Reaktionsprodukt als Farbindikator zur Anzeige einer Kühlkette in der Lebensmittelindustrie einzusetzen, wobei es bei Unterbrechung der Kühlkette zur Zersetzung des Reaktionsproduktes kommt, was mit einer Umfärbung des Indikators von intensiv-rot zu braun einhergeht.

Das Reaktionsprodukt kann zudem auch als stabile Lebensmittelfarbe, z.B. zur Färbung von Speiseeis, eingesetzt werden.

Eine besonders vorteilhafte Verfahrensvariante sei anhand des folgenden Beispiels erläutert.

Schritt A) Das bereitgestellte Ausgangsmaterial ist bei diesem Beispiel gepresster Rapskuchen, idealerweise schonend und kalt gepresst, mit typischen Restölgehalten von 20%; auch höher stellt kein Problem dar.

Schritt B) Der Kuchen wird aufgebrochen, idealerweise unmittelbar nach dem Pressen, noch warm.

Schritt C) Das Kuchengranulat wird mit Wasser dispergiert (1 Teil Kuchen und max. 6 Teile Wasser) und ist vorsichtig zu Rühren (1h).

Schritt D) Diese Dispersion ist mit Lauge auf pH 10 bis 11 einzustellen und vorsichtig zu Rühren, üblicherweise 1 h.

Schritt E) Die Dispersion aus D ist mit EtOH (vorzugsweise 30-60%ig - bezogen auf Volumenprozent) auf 12 Vol.% EtOH Konzentration zu bringen, somit wird die Wassermenge in Punkt C um das in diesem 30-60%igen EtOH enthaltene Wasser reduziert.

Schritt F) Damit lösen sich die Schalen vom Endosperm (Kotyledon) mit dem Restöl und können zentrifugal abgetrennt werden. Es entstehen ein Oberlauf und eine Schalenfraktion.

Schritt G) Ausfällung vom Protein durch Ansäuern auf vorzugsweise pH = 4,5 bis 7,2 aus dem Oberlauf (Oberlauf: Leichte Phase der Separation aus Schritt F) mit einem pH-Wert von vorzugsweise 9,7 bis 10,5) zur Trennung: Öl - Wässrige sinapinsäurehaltige Phase - Proteinkonzentratphase (Proteinquark) oder Trennung in sinapinsäurehaltige Öl/Wasserphase und Proteinkonzentratphase; Unterstützt werden kann dieser Schritt durch einer intensive Scherung, um die Ölfreisetzung zu erleichtern.

Schritt H) Abtrennung der gefällten Proteine als Quark (schwere Phase (in der Regel Feststoffphase bzw. hier Quarkphase)) und ggf. Triglyceride (als leichtes Öl) aus dem Oberlauf (leichte Phase bzw. alkoholisch-wässrige Phase), insbesondere zentrifugal.

Schritt K) Entgasen der leichten alkoholisch-wässrigen Phase
Schritt L) optionale Abtrennung einer Proteinrestphase aus der sinapinsäurehaltigen alkoholisch-wäßrigen Phase
Schritt I) Zugeben von Laccase zu der sinapinsäurehaltigen alkoholisch-wäßrigen Phase nach dem Schritte H), K) und/oder L) mit oder ohne Restölgehalt und Abwarten einer Reaktionszeit, bis sich eine rote Färbung einstellt.

Schritt M) Stabilisierung der alkoholisch-wäßrigen Phase durch Entfernung von Alkohol oder Wasser unter Einstellung eines TS-Gehalts von größer als 20%, vorzugsweise zwischen 30-35%.

Die Separation soll anhand einiger Beispiele zur besseren Veranschaulichung nachfolgend erläutert werden.

### Beispiel:

B1) Ein kalt gepresster proteinhaltiger Kuchen, welcher bis zum Schritt F verarbeitet wird, weist nach seiner Verarbeitung folgende Phasen auf: 17% schwere Phase als Schalenanteil vom Zulauf mit 20 % der Kuchen-Proteine und 83% Oberlauf als Protein-Polyphenol-Öl-Phosphatid-Phase mit 80 % der Kuchenproteine.

B2) Ein warm gepresster Kuchen, welcher bis zum Schritt F verarbeitet wird, weist nach seiner Verarbeitung folgende Phasen auf: 26% Schwere Phase als Schalenanteil vom Zulauf mit 30 % der Kuchen-Proteine und 74% Oberlauf als Protein-Polyphenol-Öl-Phosphatid-Phase mit 70 % der Kuchenproteine.

B3) Ein heiß gepresster Kuchen, welcher in Schritt F verarbeitet wird, weist nach seiner Verarbeitung folgende Phasen auf: 30% Schwere Phase als Schalenanteil vom Zulauf mit 50 % der Kuchen-Proteine und 70% Oberlauf als Protein-Polyphenol-Öl-Phosphatid-Phase mit 50 % der Kuchenproteine.

### Zu Schritt G) - Proteinfällung

Aus dem Oberlauf (Oberlauf = leichte Phase) der Separation im vorangegangenen Schritt werden die Proteine durch pH-Verschiebung auf den Bereich von 4,5 bis ca. 7 ausgefällt. Die wasserunlöslichen Proteine, welche jedoch in wässriger Lösung quellbar sind, bilden zusammen mit den ausgefallenen Globulinen die Proteinfraktion des "Proteinquarks". Die Flüssigkeit in dieser Fraktion hat dieselbe Zusammensetzung wie die Flüssigkeit der Mittelphase (Oberlauf ohne Triglyceride). Da aber die Quarkphase nur 10-30 Gew.% des Zulaufes ausmacht, (mit einem relativ hohem Anteil an Trockenmasse, 15-25 Gew.% Trockensubstanz), sind in der Quarkphase mengenmäßig auch wesentlich weniger Polyphenole zu finden als in der Mittelphase, wenngleich die Konzentration der Polyphenole bezogen auf das Wasser dieselbe ist.

Damit ist eine Proteinphase aus wasserunlöslichen jedoch gequollenen Proteinen mit Globulinen verfügbar, die mit Polyphenolen abgereichert ist. Diese Kombination aus alkalisch-ethanolischem Milieu in den Schritten A-F gefolgt von einem saueralkoholischen Milieu zur Proteinfällung stellen sehr gute Bedingungen für eine Polyphenol-Extraktion dar. Überraschenderweise hat sich für Raps (Sinapin und Derivate) hier die Beobachtung für andere Polyphenole (Tyrosol und Derivate u.a,) aus anderen Bereichen wie der Verarbeitung von Oliven bestätigt, obwohl deutlich mehr reaktionsaktive Stoffe wie Proteine und Zucker in der Suspension enthalten sind.

Damit werden Verdünnungen wie in der Literatur beschrieben hinfällig, um auf gleichwertige Polyphenol Extraktionsraten des Wässrigen zu kommen (so etwa wiederum Kroll et al, "Rapssamenproteine - Struktur, Eigenschaften, Gewinnung und Modifizierung", Deutsche Lebensmittel-Rundschau, Heft 3, 2007, S. 109.

Da das reine Triglycerid aus der Flüssigkeit als leichte Phase verdrängt wird, kann der Restölgehalt im Protein-Endprodukt auf unter 15 Gew.%, auch unter 13 Gew.% bezogen auf Trockensubstanz gesenkt werden.

Da die Temperaturen während des gesamten Prozesses <= 50 °C betragen, kann auch von einem nativen Endprodukt gesprochen werden.

Vorteilhaft ist ein Scheren des weiter zu verarbeitenden Breis vor der Phasentrennung des Schrittes H (vor der Ölabtrennung) und nach Schritt F) oder G) des Anspruchs 1 zur Verbesserung der Verdrängungsextraktion. Dieses Scheren kann mit einer Schervorrichtung, wie z.B. einem Homogenisator oder einem Intensivmischer durchgeführt werden, um derart noch mehr Öl zu gewinnen.

Das Scheren mit einer Schervorrichtung kann im kontinuierlichen Prozess durchgeführt werden. Insgesamt wird vorzugsweise ein kontinuierlicher Prozess realisiert.

In weiteren Versuchen hat sich gezeigt, dass bei einer Vorbehandlung der Schritte C), D) und E) die Sinapinsäure in der "Wasserphase" angereichert wird. Dies ist für das vorliegende Verfahren vorteilhaft. So hat die Wahl des Ausgangsmaterials einen Einfluss auf die Menge der zur Reaktion bereitstehenden Sinapinsäure.

Bei den einzelnen Versuchen wurden unterschiedliche Ansätze aus verschiedenem Rohmaterial bzw. Ausgangsmaterial plus Wasser gewählt, wobei die Proben zwar verschiedene Mengen hatten, dies wurde aber normiert bzw. geeignet umgerechnet.

Es hat sich gezeigt, dass der Polyphenolgehalt in der wässrigen Phase auf mehr als das 4-fache ansteigen kann, wenn als Ausgangsmaterial "kalt gepresster Rapspresskuchen" anstelle von "heißgepresstem Rapspresskuchen" genutzt wird. Die Verwendung des frischen Materials ist auch insoweit vorteilhaft. Denn genauso wie der Polyphenolanteil in der Wasserphase angereichert wird, wird er in der Proteinquarkphase abgereichert. So wird bei einem heiß gepressten Kuchen der Anteil von 9,4 % Polyphenolen (Trockensubstanz "TS" im Rohmaterial) auf 5,6 Gew.% TS im Proteinquark oder Quarkpulver abgereichert bzw. beim kalt gepressten Kuchen von 18,6 Gew.% TS auf 10,1 Gew.% TS im Quarkpulver. Somit ist diese Konzentration der Polyphenole bezogen auf die Trockenmasse im Feststoff nur noch etwa halb so groß wie im Ausgangsmaterial.

Damit ist eine Proteinphase aus wasserunlöslichen jedoch gequollenen Proteinen mit Globulinen verfügbar, die in Hinsicht auf den Polyphenolgehalt abgereichert worden ist. Es verbleiben in der Wasserphase ca. 55 Gew.% der Polyphenole in folgenden Konzentrationen:

| Kuchenart | Verdünnung beim Verfahren | PP in der Wasserphase (mg) | PP in der Wasserphase normiert auf eine Verdünnung 1 Teil Saat + 6 Teile Fluid |
|---|---|---|---|
| | Anteil Wasser bezogen auf 1 Anteil Kuchen | | |
| Kalt | 4,5 | 3976 | 2982 |
| Warm | 4,2 | 3183 | 2228 |
| Heiß | 6,0 | 1053 | 1058 |

Folgende Einflussfaktoren sollten bei der Verarbeitung beachtet werden: Beim Heißpressen werden Polyphenole (PP) abgebaut. Es ist gemessen worden, dass der PP-Gehalt bei kaltgepresster Saat bei 18 mg/g lag, bei heiß gepresster Saat jedoch bei 8,8 mg/g. Aus der Literatur sind ähnliche Werte bekannt (6,2 mg/g bei Jeroch et al. 1999). Neben der Reduzierung der Polyphenole im Rohmaterial geht ein Entestern des Sinapins zu Sinapinsäure vonstatten.

Durch das Kaltpressen sind nach dem o.g. Verfahren die Polyphenole mengenmäßig am Stärksten bei der Kaltpressung in die Wasserphase bzw. präziser "Polyphenol-Albumin-Phase" des Schrittes H) überführt. Sie liegen infolge der alkalischen Vorbehandlung (+Temperatur und EtOH) im Wesentlichen als Sinapinsäure oder Salze der Sinapinsäure vor und nicht mehr als Sinapin und noch nicht als Canolol.

Nunmehr ist es vorteilhaft, der Polyphenol-Albumin-Phase" des Schrittes H ein Enzym, insbesondere Laccase, zuzugeben. Als besonders vorteilhaft hat sich das Enzym "Laccase C" der ABA Spezialsysteme GmbH, Wolfenbüttel, Deutschland, erwiesen. Dieses Enzym wird vorzugweise in zumindest 0,1 g/l, vorzugsweise 0,15 bis 0,25 g/l an Laccase bezogen auf eine Enzymaktivität von 0,28 Kilounits zugegeben.

Bevorzugt umfasst zumindest 30 Gew. %, vorzugsweise über 50 Gew.%, der Trockensubstanz des Wertproduktes das Reaktionsprodukt aus Laccase und der vorgenannten natürlich gewonnenen Sinapinsäure.

Als besonders geeignet hat sich dabei zur Verarbeitung geschälter, einfach oder zweifach entölter Rapspresskuchen erwiesen. Bei einem kaltgepresstem Rapspresskuchen ist die Summe aus dem Sinapingehalt und dem Sinapinsäuregehalt größer als beim warmgepressten Rapspresskuchen. Vorteilhaft ist zudem, wenn der Anteil an Sinapinsäure in dem Sinapin- Sinapinsäuregemisch möglichst groß ist.

Es ist vorteilhaft, die Bedingungen der Schritte C) bis F) so zu wählen, dass möglichst viel Sinapinsäure entsteht. Hierzu ist es vorteilhaft, wenn der pH-Wert in Schritt D) größer als 10 ist, wenn die Verweilzeit t mindestens 30 min oder mehr beträgt und wenn die Temperatur bei mindestens T = 20° C liegt. Auch bei den Schritten E) bis H) beträgt die Temperatur vorteilhaft mindestens 20° C.

Die Abspaltung einer Cholingruppe aus dem Sinapin zu Sinapinsäure gelingt im alkalischen Milieu und bei leicht erhöhten Temperaturen besser.

Aus dem Oberlauf des Schrittes H), der Polyphenol-Albuminphase mit einem Sinapin-/Sinapinsäureanteil, einem braunen Fluid mit zumindest beispielsweise ca. 8% TS, davon 2% Protein (85% dessen wiederum wasserlösliches Napin) und ca. 6-7 % Zucker und etwas ölhaltigen Substanzen wird ein rotes Fluid gewonnen.

Danach wird dem Oberlauf (vorzugsweise bei Raumtemperatur) das Enzym Laccase in der Menge von 0,1g von einer Lösung mit 10.000 Units (Menge 1 g) also in etwa 1.000 Units zugegeben. Sodann erfolgt eine Belüftung für wenigstens 30 min, vorzugsweise ca. 1 Stunde. Bereits 1 + 1 (1kUnit) bewirken ein Ergebnis. Nachdem eine genügende Reaktionszeit T verstrichen ist, wird der Oberlauf, dem Laccase zugegeben worden ist, rot. Es entsteht ein rotes Fluid, das eine Färbung nach Art der Farben RAL 3004, 3005 und/oder 3006 aufweist. Die entstehende Farbe ähnelt der Farbe der Frucht "rote Beete". Das entstehende Fluid ist vielfach verwendbar, so als Lebensmittelzusatz zum Färben nach Art einer Farbe des Typs "rote Beete.".

Das Wertprodukt kann ein Wertprodukt sein, welches zusätzlich zum Reaktionsprodukt noch Restprotein (Albumin/Napin) aufweist oder es kann ein Wertprodukt sein, welches durch eine zusätzliche Separation 140 von diesem Restprotein weitestgehend befreit ist.

Die sinapinsäurehaltige wäßrige und/oder alkoholische Phase (110 oder 160) kann aus kaltgepressten Saaten und/oder Früchten hergestellt sein.

Der pH-Wert der wäßrigen und/oder alkoholischen Phase (110 oder 160) kann pH=7 oder weniger betragen.

Die wäßrige und/oder alkoholische Phase (110 oder 160) kann einen Trockensubstanzgehalt vor der Zugabe von Laccase (130) von weniger als 3%, vorzugsweise weniger als 1% aufweisen.

Das wasserlösliche organische Lösemittel kann ein linearer aliphatischer Alkohol sein.

Das erfindungsgemäße Wertprodukt ist vorzugsweise nach einem erfindungsgemäßen Verfahren hergestellt.

### Bezugszeichen

- 10: Rapspresskuchen
- 20: Mischen
- 30: Ethanol
- 40: Wasser
- 50: Lauge
- 60: Separation
- 70: Schalen
- 80: Salzsäure-Lösung
- 90: Separation
- 100: Proteinquark
- 110: Sinapinsäurehaltige Phase
- 120: H2O + EtOH - Entfernung
- 130: Laccase
- 140: Separation
- 150: Restprotein
- 160: Lösung und/oder Dispersion
- 170: H2O + EtOH - Entfernung
- 180: Wertprodukt (mehr als 30 % TS)

## Patentansprüche

1. Verfahren zur Gewinnung einer Wertstoffphase, insbesondere Wertproduktes aus einem nativen Stoffgemenge, mit folgenden Schritten:
- Schritt A: Bereitstellen des nativen Stoffgemenges aus Saaten von Kreuzblütengewächsen (Brassicaceae), mit einem Anteil von harten, zerbrechbaren Schalen oder in geschälter Form, insbesondere von Rapssaaten als Stoffgemenge aus den vollständigen Saaten oder aus bereits (teil-) entölten Saaten, insbesondere als Presskuchen, der bei einem Abpressen von Öl insbesondere mit einer Presse als Rückstand der Ölgewinnung verbleibt;
- Schritt B: sofern das Stoffgemenge aus Schritt A noch nicht zerkleinert ist: Zerkleinern des Stoffgemenges, wobei ggf. die Schalen aufgebrochen werden;
- Schritt C: Dispergieren und/oder Mischen (20) des zerkleinerten Stoffgemenges aus Schritt A) oder B) mit Wasser (40), wobei auf einen Teil zerkleinertes Stoffgemenge vorzugsweise bis zu maximal 8, besonders vorzugsweise bis zu maximal 6, insbesondere bis zu maximal 5 Teile Wasser zugegeben werden und wobei das Wasser (40) und das zerkleinerte Stoffgemenge gerührt werden, so dass sich ein fließfähiger Brei bzw. eine Dispersion ergibt;
- Schritt D: Einstellen des pH-Wertes des Breis aus Schritt C) in einen alkalischen Bereich pH > 9, 5;
- Schritt E: Zugeben eines wasserlöslichen organischen Lösemittels, vorzugsweise von Ethanol (30), insbesondere in mit Wasser verdünnter Form, zu dem Brei aus Schritt D) im Anschluss an das Einstellen des pH-Wertes des Breis im Schritt D; insbesondere derart, dass eine Alkoholkonzentration erreicht wird, die kleiner als 30% ist, um die Schalen vom Endosperm der Saaten/Früchte zu lösen;
- Schritt F: Abtrennen einer Feststoffphase (60), welche den überwiegenden Anteil der ggf. noch vorhandenen Schalen (70) aufweist, vorzugsweise in einer Zentrifuge im Zentrifugalfeld;
- Schritt G: Verschieben des pH-Wertes des Feststoffphase befreiten Breis aus Schritt F) in den pH-Bereich von pH = 4,5 bis pH = 7,2; und
- Schritt H: Trennen des schalenfreien Breis (90), dessen pH-Wert in Schritt G) ins Saure verschoben worden ist, vorzugweise in einer Zentrifuge, insbesondere in wenigstens einem Dekanter oder einem Separator, in mehrere Phasen, wobei zumindest eine dieser Phasen eine sinapinsäurehaltige Phase (110) ist;
- Schritt I: Zugabe von Laccase (130) zu der sinapinsäurehaltigen Phase (110) des Schrittes H) direkt oder nach einem Durchlaufen weiterer Zwischenschritte.

2. Verfahren nach Anspruch 1, **dadurch gekennzeichnet, dass** die sinapinsäurehaltige Phase (110) des Schrittes I) eine Flüssigphase ist, der Laccase zugesetzt worden ist und die nach einer Reaktionszeit von maximal 30 min eine rote Färbung annimmt.

3. Verfahren nach Anspruch 1 oder 2, **dadurch gekennzeichnet, dass** der sinapinsäurehaltigen Phase (110) des Schrittes H) im Schritt I) Laccase in Anwesenheit von Sauerstoff in folgender Menge zugesetzt wird: zumindest 0,1 g/l, vorzugsweise 0,15 bis 0,25 g/l an Laccase (130) bezogen auf eine Enzymaktivität von 0,28 Kilounits, unter Bildung des Reaktionsproduktes gemäß Anspruch 1.

4. Verfahren nach Anspruch 1 oder 2, **dadurch gekennzeichnet, dass** im Schritt H) folgende Phasentrennung in einem oder zwei Schritten, vorzugsweise in einer Zentrifuge, insbesondere in einem Dekanter oder Separator, vorgenommen wird:
- ölhaltige Phase (190) mit Triglyceridgehalt;
- wässrige Phase (110) mit Albumin und Sinapinsäuregehalt; und
ggf. eine dritte Phase (100) mit einem weiteren Wertprodukt.

5. Verfahren nach einem der vorhergehenden Ansprüche, **dadurch gekennzeichnet, dass** im Schritt H) folgende Phasentrennung in einem oder zwei Schritten, vorzugsweise in einer Zentrifuge, insbesondere in einem Dekanter oder Separator, in zwei Wertstoffphasen vorgenommen wird, mit zumindest einer wässrigen Phase (110) mit Albumingehalt und Sinapinsäuregehalt und Restölgehalt.

6. Verfahren nach einem der vorstehenden Ansprüche, **dadurch gekennzeichnet, dass** das Stoffgemenge/Ausgangsmaterial (10) als "kurz zuvor hergestelltes Zwischenprodukt" verarbeitet wird, d.h. nach der Vorstufe sind nicht mehr als 31 Tage, vorzugsweise nicht mehr als 3 Tage, besonders vorzugsweise weniger als 48 Stunden, insbesondere weniger als 24 Stunden, vergangen.

7. Verfahren nach einem der vorstehenden Ansprüche, **dadurch gekennzeichnet, dass** als das Stoffgemenge in Schritt A kalt gepressten Material, insbesondere ein kalt gepresster Rapspresskuchen (10) verwendet wird, der bei einer Temperatur kleiner als 70°C, besonders vorzugsweise sogar kleiner als 60°C, gepresst worden ist.

8. Verfahren nach einem der vorstehenden Ansprüche, **dadurch gekennzeichnet, dass** einer oder mehrere der Abtrennungsschritte in einem 3-Phasendekanter oder in zumindest zwei Schritten in 2-Phasendekantern erfolgt/erfolgen, insbesondere in einem Düsenseparator erfolgt/erfolgen.

9. Verfahren nach einem der vorhergehenden Ansprüchen, **dadurch gekennzeichnet, dass** der Gehalt an wasserlöslichem organischen Lösemittel im wässrigen Anteil des Breis (I) nach dem Zugeben des wasserlöslichen organischen Lösemittels weniger als 45 Vol. %, vorzugsweise weniger als 30 Vol %, und besonders vorzugsweise weniger als 15 Vol %, beträgt.

10. Verfahren nach einem der vorhergehenden Ansprüche, **dadurch gekennzeichnet, dass** die Temperatur während sämtlicher Verfahrensschritte, wozu nicht das Pressen zur Erzeugen des Presskuchens gehört, unterhalb von 60 °C, insbesondere unterhalb von 50°C, liegt.

11. Wertprodukt (180) hergestellt nach einem Verfahren nach Anspruch 4, in Form eines flavonoid-enthaltenden Phenolgemisches umfassend ein Reaktionsprodukt, welches gebildet wird bei der Zugabe von Laccase (130) zu einer sinapinsäurehaltigen wäßrigen und/oder alkoholischen Phase (110 oder 160) hergestellt aus Pflanzen und/oder Pflanzenteilen, vorzugsweise aus Saaten und/oder Früchten von Kreuzblütengewächsen (Brassicaceae), insbesondere von Rapsfrüchten oder Camelina, unter Anwesenheit von Sauerstoff, wobei die wäßrige und/oder alkoholische Phase (110, 160) nach einer erfolgten Abtrennung einer Schalenfraktion, einer Ölfraktion und einer Proteinkonzentratfraktion, als drittes Wertprodukt gemäß Anspruch 4, nach dem Durchführen der Schritte A-H, bereitgestellt wird.

12. Wertprodukt nach Anspruch 11, **dadurch gekennzeichnet, dass** das Wertprodukt (180) als eine wäßrige und/oder alkoholische Lösung und/oder Dispersion vorliegt, in welcher das Reaktionsprodukt gelöst und/oder dispergiert vorliegt.

13. Wertprodukt nach Anspruch 12, **dadurch gekennzeichnet, dass** die alkoholische und/oder wäßrige Lösung und/oder Dispersion einen Trockensubstanzgehalt von mehr als 30 %, vorzugsweise mehr als 55 % aufweist.

14. Wertprodukt nach einem der vorhergehenden Ansprüche, **dadurch gekennzeichnet, dass** das Reaktionsprodukt eine Phenolische Verbindung, insbesondere ein Flavonoid, ist.

15. Wertprodukt nach einem der vorhergehenden Ansprüche, **dadurch gekennzeichnet, dass** das Wertprodukt (180) weniger als 500 mg/kg, vorzugsweise weniger als 400 mg/kg, an Sinapinsäure aufweist und insbesondere das Wertprodukt (180) mehr Mol-% an Reaktionsprodukt als an Sinapinsäure aufweist.

## Claims

1. Method for obtaining a valuable material phase, in particular a valuable product from a native mixture of substances, comprising the following steps of:
- step A: providing the native mixture of substances from seeds of cruciferous plants (Brassicaceae), having a proportion of hard, breakable hulls or in hulled form, in particular from rapeseed as a mixture of substances from the complete seeds or from seeds which have already been (partially) deoiled, in particular as a press cake which remains as a residue from oil extraction when oil is pressed off, in particular using a press;
- step B: if the mixture of substances from step A has not yet been comminuted: comminution of the mixture of substances, wherein the hulls may optionally be broken up;
- step C: dispersing and/or mixing (20) the comminuted mixture of substances from step A) or B) with water (40), wherein preferably up to a maximum of 8, particularly preferably up to a maximum of 6, in particular up to a maximum of 5 parts of water is added to one part of comminuted mixture of substances, and wherein the water (40) and the comminuted mixture of substances are stirred so that a flowable slurry or dispersion is obtained;
- step D: adjusting the pH value of the slurry from step C) to an alkaline range pH value >9.5;
- step E: adding a water-soluble organic solvent, preferably ethanol (30), in particular in a form diluted with water, to the slurry from step D) following the adjustment of the pH value of the slurry in step D; in particular in such a way that an alcohol concentration is achieved which is less than 30% in order to dissolve the hulls from the endosperm of the seeds/fruits;
- step F: separation of a solid phase (60) which comprises the majority of the hulls (70) which may optionally still be present, preferably in a centrifuge in a centrifugal field;
- step G: shifting the pH value of the pulp freed from the solid phase from step F) into the pH range from pH = 4.5 to pH = 7.2; and
- step H: separating the hull-free pulp (90), the pH value of which has been shifted to the acidic range in step G), preferably in a centrifuge, in particular in at least one decanter or a separator, into a plurality of phases, wherein at least one of these phases is a sinapinic acid-containing phase (110);
- step I: addition of laccase (130) to the sinapinic acid-containing phase (110) of step H) directly or after passing through further intermediate steps.

2. Method according to claim 1, **characterized in that** the sinapinic acid-containing phase (110) of step I) is a liquid phase to which laccase has been added and which takes on a red color after a reaction time of at most 30 min.

3. Method according to claim 1 or 2, **characterized in that** laccase is added to the sinapinic acid-containing phase (110) of step H) in step I) in the presence of oxygen in the following amount: at least 0.1 g/l, preferably 0.15 to 0.25 g/l of laccase (130), based on an enzyme activity of 0.28 kilounits, to form the reaction product according to claim 1.

4. Method according to claim 1 or 2, **characterized in that** in step H) the following phase separation is carried out in one or two steps, preferably in a centrifuge, in particular in a decanter or separator:
- oily phase (190) with triglyceride content;
- aqueous phase (110) with albumin and sinapinic acid content; and optionally a third phase (100) with a further valuable product.

5. Method according to one of the preceding claims, **characterized in that** in step H) the following phase separation is carried out in one or two steps, preferably in a centrifuge, in particular in a decanter or separator, into two valuable product phases, with at least one aqueous phase (110) with albumin content and sinapinic acid content and residual oil content.

6. Method according to one of the preceding claims, **characterized in that** the mixture of substances/starting material (10) is processed as a "recently produced intermediate", i.e. no more than 31 days, preferably no more than 3 days, particularly preferably less than 48 hours, in particular less than 24 hours, have elapsed after the preliminary stage.

7. Method according to one of the preceding claims, **characterized in that** cold-pressed material, in particular a cold-pressed rapeseed press cake (10), which has been pressed at a temperature of less than 70°C, particularly preferably even less than 60°C, is used as the mixture of substances in step A.

8. Method according to one of the preceding claims, **characterized in that** one or more of the separation steps is/are carried out in a 3-phase decanter or in at least two steps in 2-phase decanters, in particular in a nozzle separator.

9. Method according to one of the preceding claims, **characterized in that** the content of water-soluble organic solvent in the aqueous portion of the slurry (I) after the addition of the water-soluble organic solvent is less than 45% by volume, preferably less than 30% by volume, and particularly preferably less than 15% by volume.

10. Method according to one of the preceding claims, **characterized in that** the temperature during all method steps, which does not include pressing to produce the press cake, is below 60°C, in particular below 50°C.

11. Valuable product (180) produced by a method according to claim 4, in the form of a flavonoid-containing phenolic mixture comprising a reaction product which is formed on the addition of laccase (130) to a sinapinic acid-containing aqueous and/or alcoholic phase (110 or 160) produced from plants and/or plant parts, preferably from seeds and/or fruits of cruciferous plants (Brassicaceae), in particular from rapeseed fruits or camelina, in the presence of oxygen, wherein the aqueous and/or alcoholic phase (110, 160) is provided as the third valuable product according to claim 4 after separation of a hull fraction, an oil fraction and a protein concentrate fraction, after steps A-H have been carried out.

12. Valuable product according to claim 11, **characterized in that** the valuable product (180) is present as an aqueous and/or alcoholic solution and/or dispersion in which the reaction product is dissolved and/or dispersed.

13. Valuable product according to claim 12, **characterized in that** the alcoholic and/or aqueous solution and/or dispersion has a dry substance content of more than 30%, preferably more than 55%.

14. Valuable product according to one of the preceding claims, **characterized in that** the reaction product is a phenolic compound, in particular a flavonoid.

15. Valuable product according to one of the preceding claims, **characterized in that** the valuable product (180) has less than 500 mg/kg, preferably less than 400 mg/kg, of sinapinic acid and, in particular, the valuable product (180) has more mol% of reaction product than of sinapinic acid.

## Revendications

1. Procédé pour l'obtention d'une phase de matière valorisée, en particulier d'un produit valorisé à partir d'un volume de matière natif, comprenant les étapes suivantes :
- étape A : préparation du volume de matière natif à partir de graines de crucifères (Brassicaceae), contenant une part de coques dures brisables ou sous une forme émondée, en particulier de graines de colza constituant le volume de matière composé des graines entières ou de graines déjà (partiellement) déshuilées, en particulier sous la forme de tourteau de pressage, constituant un résidu de l'extraction d'huile après un pressage de l'huile, en particulier à l'aide d'une presse ;
- étape B : si le volume de matière de l'étape A n'est pas encore broyé, broyage du volume de matière, en ouvrant si nécessaire les coques ;
- étape C : dispersion et/ou mélange (20) du volume de matière broyé issu de l'étape A) ou B) avec de l'eau (40), une part de volume de matière broyé étant de préférence additionnée de jusqu'à 8 parts au maximum, en particulier jusqu'à 6 parts au maximum, notamment jusqu'à 5 parts au maximum d'eau, et l'eau (40) et le volume de matière broyé étant remués de façon à obtenir une bouillie fluide ou une dispersion ;
- étape D : ajustement du pH de la bouillie de l'étape C) dans la plage alcaline de pH > 9,5 ;
- étape E : ajout d'un solvant organique hydrosoluble, de préférence d'éthanol (30), en particulier dilué avec de l'eau, à la bouillie issue de l'étape D) après l'ajustement du pH de la bouillie dans l'étape D, en particulier de façon à atteindre une concentration d'alcool inférieure à 30 %, afin de détacher les coques de l'endosperme des graines/fruits ;
- étape F : séparation d'une phase solide (60) contenant la majeure partie des coques restantes (70), de préférence par centrifugation en champ centrifuge ;
- étape G : décalage du pH de la bouillie débarrassée de la phase solide issue de l'étape F) dans la plage de pH = 4,5 à pH = 7,2 et
- étape H : séparation de la bouillie débarrassée des coques (90), dont le pH a été décalé dans la plage acide dans l'étape G), en plusieurs phases dont une au moins est une phase contenant de l'acide sinapique (110), de préférence dans une centrifugeuse, en particulier dans au moins un décanteur ou un séparateur ;
- étape I : ajout de laccase (130) à la phase contenant de l'acide sinapique (110) issue de l'étape H), immédiatement ou après l'exécution d'autres étapes intermédiaires.

2. Procédé selon la revendication 1, **caractérisé en ce que** la phase contenant de l'acide sinapique (110) de l'étape I) est une phase liquide qui a été additionnée de laccase et qui prend une teinte rouge après un temps de réaction de 30 minutes au maximum.

3. Procédé selon la revendication 1 ou 2, **caractérisé en ce que** la phase contenant de l'acide sinapique (110) de l'étape H) est additionnée de laccase dans l'étape I) en présence d'oxygène, dans les quantités suivantes : au moins 0,1 g/l, de préférence 0,15 à 0,25 g/l de laccase (130) par rapport à une activité enzymatique de 0,28 kilo-unités, en formant le produit de réaction selon la revendication 1.

4. Procédé selon la revendication 1 ou 2, **caractérisé en ce que** la séparation de phases suivante est exécutée dans l'étape H) en une ou deux étapes, de préférence dans une centrifugeuse, en particulier dans un décanteur ou un séparateur :
- phase huileuse (190) contenant des triglycérides,
- phase aqueuse (110) contenant de l'albumine et de l'acide sinapique, et éventuellement une troisième phase (100) contenant un autre produit valorisé.

5. Procédé selon l'une des revendications précédentes, **caractérisé en ce que** la séparation de phases suivante est exécutée dans l'étape H) en une ou deux étapes, en deux phases de matière valorisée comprenant au moins une phase aqueuse (110) contenant de l'albumine et contenant de l'acide sinapique et contenant un résidu d'huile, de préférence dans une centrifugeuse, en particulier dans un décanteur ou un séparateur.

6. Procédé selon l'une des revendications précédentes, **caractérisé en ce que** le volume de matière/la matière de départ (10) est traitée comme un « produit intermédiaire produit peu de temps auparavant », c'est-à-dire pas plus de 31 jours, de préférence pas plus de 3 jours, en particulier moins de 48 heures, notamment moins de 24 heures après l'étape antérieure.

7. Procédé selon l'une des revendications précédentes, **caractérisé en ce que** le volume de matière utilisé dans l'étape A est une matière pressée à froid, en particulier un tourteau de colza (10) pressé à froid, qui a été pressé à une température inférieure à 70 °C, voire de préférence inférieure à 60 °C.

8. Procédé selon l'une des revendications précédentes, **caractérisé en ce qu'**une ou plusieurs des étapes de séparation sont exécutées dans un décanteur à trois phases ou en au moins deux étapes dans des décanteurs à deux phases, en particulier dans un séparateur à buses.

9. Procédé selon l'une des revendications précédentes, **caractérisé en ce que** la teneur en solvant organique hydrosoluble dans la fraction aqueuse de la bouillie (I) après l'ajout du solvant organique hydrosoluble est inférieure à 45 % du volume, de préférence inférieure à 30 % du volume et en particulier inférieure à 15 % du volume.

10. Procédé selon l'une des revendications précédentes, **caractérisé en ce que** la température est inférieure à 60 °C, en particulier inférieure à 50 °C, pendant toutes les étapes de procédé, qui n'incluent pas le pressage pour la production du tourteau.

11. Produit valorisé (180) fabriqué par un procédé selon la revendication 4, sous la forme d'un mélange de phénols contenant des flavonoïdes incluant un produit de réaction formé lors de l'ajout de laccase (130) à une phase aqueuse et/ou alcoolique contenant de l'acide sinapique (110 ou 160) fabriquée à partir de plantes et/ou de parties de plantes, en particulier de graines et/ou de fruits de crucifères (Brassicaceae), en particulier de fruits de colza ou de cameline, en présence d'oxygène, la phase aqueuse et/ou alcoolique (110, 160) étant produite après séparation d'une fraction de coques, d'une fraction d'huile et d'une fraction de protéines concentrée, pour former le troisième produit valorisé selon la revendication 4 après l'exécution des étapes A à H.

12. Produit valorisé selon la revendication 11, **caractérisé en ce que** le produit valorisé (180) se présente comme une solution et/ou dispersion aqueuse et/ou alcoolique dans laquelle le produit de réaction est présent en solution et/ou en dispersion.

13. Produit valorisé selon la revendication 12, **caractérisé en ce que** la solution et/ou dispersion aqueuse et/ou alcoolique présente un taux de matière sèche supérieur à 30 %, de préférence supérieur à 55 %.

14. Produit valorisé selon l'une des revendications précédentes, **caractérisé en ce que** le produit de réaction est un composé phénolique, en particulier un flavonoïde.

15. Produit valorisé selon l'une des revendications précédentes, **caractérisé en ce que** le produit valorisé (180) contient moins de 500 mg/kg, de préférence moins de 400 mg/kg d'acide sinapique et le produit valorisé (180) contient en particulier un pourcentage molaire plus élevé de produit de réaction que d'acide sinapique.
